# EUROPEAN PATENT APPLICATION

(11) **EP 4 390 249 A1**
(43) Date of publication of application: **26.06.2024**
(21) Application number: 23217845.9
(22) Date of filing: 19.12.2023
(51) Int. Cl.: F24F 6/02, F24F 6/08, F24F 8/22, F24F 6/00

(54) **HUMIDIFIER**

(30) Priority: 19.12.2022 KR 20220177801
(71) Applicant: LG Electronics Inc., Yeongdeungpo-gu Seoul 07336 (KR)
(72) Inventor: Choi, Chiyoung, Geumcheon-gu, Seoul 08592 (KR); Choi, Jaeheuk, Geumcheon-gu, Seoul 08592 (KR); Park, Dongryul, Geumcheon-gu, Seoul 08592 (KR); Kim, Yongmin, Geumcheon-gu, Seoul 08592 (KR); Lee, Jeonghoon, Geumcheon-gu, Seoul 08592 (KR); Moon, Sunyoung, Geumcheon-gu, Seoul 08592 (KR); Hong, Seongkyeol, Geumcheon-gu, Seoul 08592 (KR)
(74) Representative: Ter Meer Steinmeister & Partner

(57) **Abstract**

The present disclosure relates to a humidifier. A humidifier according to one aspect of the present disclosure includes: a case; and a humidification assembly disposed inside the case, that produces mist, wherein the humidification assembly includes: a humidification chamber with a space for holding water; a sterilizer that irradiates the inside of the humidification chamber with light; and a reflector disposed along a peripheral wall of the humidification chamber. Thus, the hygiene of the humidification assembly can be maintained easily.

## Description

### BACKGROUND

### Field

The present disclosure relates to a humidifier, and more particularly, to a humidifier that supplies clean humidified air.

### Related Art

A humidifier is an appliance that supplies moisture-containing air within a room. The humidifier is equipped with a water reservoir for storing water, and is classified as ultrasonic humidifier, heated humidifier, natural evaporative humidifier, or hybrid humidifier depending on the method of humidification.

Ultrasonic humidifiers are equipped with a vibrator to create vibrations from ultrasonic waves, and convert water into fine particles using the vibrations from the vibrator and spray them.

Ultrasonic humidifiers have advantages such as converting the water stored in the water reservoir into fine particles and spraying large amounts of mist.

However, conventional humidifiers are problematic in that the device may have a poor state of cleanliness because of the stagnant water in the device. As such, air sprayed from the device can be highly contaminated.

Another problem with conventional humidifiers is that, even if an ultraviolet sterilizer is installed inside the device, ultraviolet light produced by the sterilizer is not evenly diffused over the entire area of the humidification device and therefore cannot uniformly sterilize the inside of the humidification device.
[Prior Art] (Document 0001) KR Patent Document 10-2022-0105488, (Document 0002) KR Patent Document 10-2018-0094813

### SUMMARY

The present disclosure is directed to overcoming the above problems and others.

Another aspect of the present disclosure is to provide a humidifier that delivers better cleanliness efficiency.

Yet another aspect of the present disclosure is to provide a humidifier that makes hygiene control easier.

A further aspect of the present disclosure is to provide a humidifier that delivers better sterilization efficiency.

A further aspect of the present disclosure is to diffuse ultraviolet light uniformly through the inside of a humidification device.

A further aspect of the present disclosure is to allow for easy installation of a sterilizer.

The aspects of the present disclosure are not limited to the foregoing, and other aspects not mentioned herein will be able to be clearly understood by those skilled in the art from the following description.

The object is solved by the features of the independent claims. Preferred embodiments are given in the dependent claims.

To accomplish the foregoing aspects, a humidifier according to an aspect of the present disclosure includes: a case.

The humidifier includes a humidification assembly disposed inside the case, that produces mist.

The humidification assembly includes a humidification chamber with a space for holding water.

The humidification assembly includes a sterilizer that irradiates the inside of the humidification chamber with light.

The humidification assembly includes a reflector disposed along a peripheral wall of the humidification chamber.

The reflector may be spaced from the sterilizer and disposed to face the sterilizer.

The humidification chamber may include an inlet through which air is admitted into the humidification chamber.

The humidification chamber may include an outer wall attached to the inlet.

The humidification chamber may include an inner wall spaced from the outer wall.

The humidification chamber may include a first side wall connecting the outer wall and the inner wall.

The sterilizer may be disposed on the first side wall.

The reflector may include an outer reflector disposed on the outer wall.

The reflector may include an inner reflector disposed on the inner wall.

The humidification chamber may include a second side wall spaced from the first side wall.

The outlet may include a supporter that extends downward toward the inside of the humidification chamber and is disposed more adjacent to the second side wall than to the first side wall.

The inner wall may include a wall surface portion spaced from the first side wall.

The inner wall may include a first edge portion that connects the wall surface portion and the first side wall and is curved from the wall surface portion in a direction away from the inside of the humidification chamber.

The inner wall may include a second edge portion being symmetrical to the first edge portion with respect to the wall surface portion.

The sterilizer may be spaced from both the inlet and the outlet.

The humidification assembly may include a heating device that heats water and communicates with the humidification chamber.

The humidification assembly may include a body including a first opening communicating with the heating device and a second opening communicating with the humidification chamber.

The sterilizer may be positioned lower than the second opening.

The sterilizer may be positioned lower than the outlet.

The outlet may include an outlet lower end positioned inside the humidification chamber.

The outlet may include a recess formed by indenting the outlet lower end upward.

The sterilizer may be positioned lower than the recess.

The outlet may include an outlet body attached to the top of the humidification chamber.

The outlet may include a mist discharge opening protruding upward from the outlet body.

The outlet may include a mist intake opening protruding from the outlet body into the humidification chamber.

The sterilizer may be positioned lower than the mist intake opening.

The humidification assembly may include a connector connecting the heating chamber and the humidification chamber.

The humidification assembly may include a mist inlet opening extending vertically toward the inside of the humidification chamber.

The humidification assembly may include a communicating opening formed through the mist inlet opening, that opens toward the connector.

The sterilizer may be positioned lower than the communicating opening.

The reflector may include a dimple formed by indenting the reflector in a direction away from the internal space of the humidification chamber.

The reflector may be made of a chrome-plated material.

The humidifier may further include an intake opening through which air is admitted into the case.

The humidifier may further include a discharge opening through which air admitted through the intake opening flows.

The humidifier may further include a humidification flow path connecting the humidification assembly and the discharge opening.

The humidifier may further include a air flow path separated from the humidification flow path and connected to the discharge opening.

Specific details of other embodiments are included in the detailed description and the drawings.

According to at least one of the embodiments of the present disclosure, the hygiene of the humidification assembly can be improved by installing a sterilizer to the humidification assembly.

According to at least one of the embodiments of the present disclosure, the hygiene of the humidification assembly can be maintained easily by irradiating the inside of the humidification device which has high humidity with ultraviolet light.

According to at least one of the embodiments of the present disclosure, ultraviolet light can be easily diffused within the humidification device by installing a reflector to the humidification device.

According to at least one of the embodiments of the present disclosure, ultraviolet light can be diffused uniformly through the inside of the humidification device by disposing a reflector along a peripheral wall of the humidification device.

The effects of the present disclosure are not limited to the foregoing, and other effects not mentioned herein will be able to be clearly understood by those skilled in the art from the following description.

### BRIEF DESCRIPTION OF THE DRAWINGS

FIG. 1 is a perspective view of a humidifier according to an embodiment of the present disclosure.
FIG. 2 is an exploded view of a humidifier according to an embodiment of the present disclosure.
FIG. 3 is a cross-sectional view of a humidifier of an embodiment of the present disclosure.
FIG. 4 is an enlarged cross-sectional view of a humidifier of an embodiment of the disclosure.
FIG. 5 shows part of a humidifier according to an embodiment of the present disclosure.
FIG. 6 is a partial cutaway view of a humidifier according to an embodiment of the disclosure.
FIG. 7 shows part of a humidifier according to an embodiment of the present disclosure.
FIG. 8 shows part of a humidifier according to an embodiment of the present disclosure.
FIG. 9 is a partial cross-sectional view of a humidifier of an embodiment of the disclosure.
FIG. 10 is a partial cross-sectional view of a humidifier of an embodiment of the present disclosure.
FIG. 11 is a graph illustrating the sterilization efficiency of a humidifier according to an embodiment of the present disclosure.
FIG. 12 is a diagram illustrating an effect according to an embodiment of the present disclosure.
FIG. 13 is a diagram illustrating an effect according to an embodiment of the present disclosure.
FIG. 14 is a diagram illustrating an effect according to an embodiment of the present disclosure.

### DESCRIPTION OF EXEMPLARY EMBODIMENTS

Hereinafter, the embodiments disclosed in the present specification will be described in detail with reference to the accompanying drawings. The same or similar elements will be assigned the same reference numerals irrespective of the reference numerals, and redundant descriptions thereof will be omitted.

The suffixes "module" and "unit" for elements used in the following embodiments are given or interchangeably used in consideration of only the ease of drafting the specification and do not have a meaning or role distinct from each other.

In describing the embodiments disclosed in the present specification, a detailed description of a related known technology will be omitted when it is deemed that it may unnecessarily obscure the subject matter of the present disclosure. Also, it should be understood that the appended drawings are intended only to help understand the embodiments disclosed in the present specification and do not limit the technical idea disclosed in the present disclosure; rather, it should be understood that all changes, equivalents, and substitutions included in the technical scope of the present disclosure are included.

Terms such as 'first', 'second', etc., may be used to describe various components, but the components are not to be construed as being limited to the terms. The terms are used only to distinguish one component from another component.

It is to be understood that when one element is referred to as being "connected to" or "coupled to" another element, it may be connected directly to or coupled directly to another element or be connected to or coupled to another element, having the other element intervening therebetween. On the other hand, it is to be understood that when one element is referred to as being "connected directly to" or "coupled directly to" another element, it may be connected to or coupled to another element without the other element intervening therebetween.

The singular expressions may include plural expressions unless the context clearly dictates otherwise.

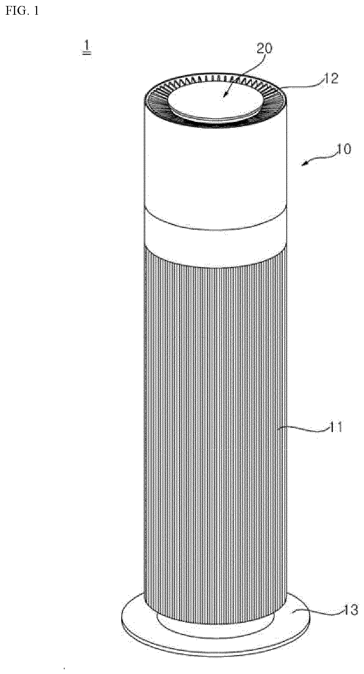

Referring to FIG. 1, a humidifier 1 will be described.

FIG. 1 is a perspective view of the humidifier 1.

The humidifier 1 may include a case 10. The case 10 may internally have a space. The case 10 may be cylindrical.

The humidifier 1 may include an intake opening 11. The intake opening 11 may be formed around the circumference of the case 10. Air from outside the humidifier 1 may enter the case 10 through the intake opening 11.

The humidifier 1 may include a discharge opening 12. The discharge opening 12 may be formed on the top of the case 10. Air drawn into the case 10 may be discharged out of the case 10 through the discharge opening 12.

The humidifier 1 may include a base 13. The base 13 may be disposed at the bottom of the case 10. The base 13 may have a larger diameter than the case 10.

The humidifier 1 may include a water reservoir 20. The water reservoir 20 may be disposed inside the case 10. A space for storage of water may be formed inside the water reservoir 20. The discharge opening 12 may be formed radially outward from the water reservoir 20. The discharge opening 12 may be formed to surround the water reservoir 20.

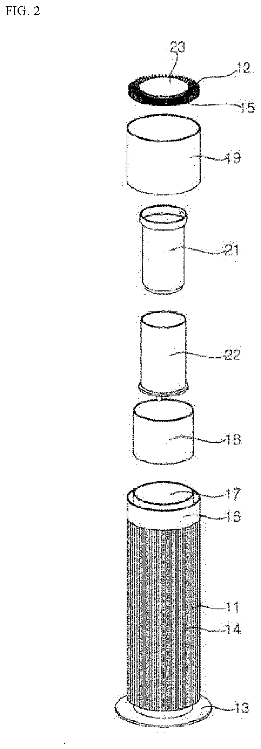

Referring to FIG. 2, the humidifier 1 will be described.

FIG. 2 is an exploded view of the humidifier 1.

The humidifier 1 may include an intake grille 14. The intake grille 14 may be disposed around the circumference of the case 10. The intake grille 14 may form the intake opening 11. The intake grille 14 may be cylindrical.

The humidifier 1 may include a discharge grille 15. The discharge grille 15 may be disposed at an upper part of the case 10. The discharge grille 15 may form the discharge opening 12. The discharge grille 15 may be ring-shaped.

The humidifier 1 may include a first outer case 16. The first outer case 16 may be cylindrical. The intake grille 14 may be attached to and detached from the first outer case 16. The first outer case 16 may form the exterior of the case 10.

The humidifier 1 may include a first inner case 17. The first inner case 17 may be cylindrical. The first inner case 17 may be disposed radially inward from the first outer case 16. The first outer case 16 and the first inner case 17 may be radially spaced apart from each other

The humidifier 1 may include a second outer case 19. The second outer case 19 may be cylindrical. The second outer case 19 may be disposed above the first outer case 16. The second outer case 19 may form the exterior of the case 10.

The humidifier 1 may include a second inner case 18. The second inner case 18 may be cylindrical. The second inner case 18 may be disposed above the first inner case 17. The second inner case 18 may be disposed radially inward from the second outer case 19. The second outer case 19 and the second inner case 18 may be radially spaced apart from each other.

The first outer case 16 and the second outer case 19 may be removably coupled together. Then again, the first outer case 16 and the second outer case 19 may be formed as a single body. The first outer case 16 and the second outer case 19 may be called "outer case".

The first inner case 17 and the second inner case 18 may be removably coupled together. Then again, the first inner case 17 and the second inner case 18 may be formed into a single body. The first inner case 17 and the second inner case 18 may be called "inner case".

The humidifier 1 may include a bucket 21. The bucket 21 may be cylindrical with an open top. The bucket 21 may internally have a space for storing water.

The humidifier 1 may include a bucket housing 22. The bucket housing 22 may surround the bucket 21. The bucket housing 22 may be cylindrical with an open top. The bucket 21 may be disposed inside the bucket housing 22.

The humidifier 1 may include a bucket cover 23. The bucket cover 23 may be disposed above the bucket 21. The bucket cover 23 may be removably attached to the discharge grille 15.

The water reservoir 20 may include the bucket 21, the bucket housing 22, and the bucket cover 23. The water reservoir 20 may be disposed inside the inner case 18. The water reservoir 20 and the inner case 18 may be radially spaced apart from each other.

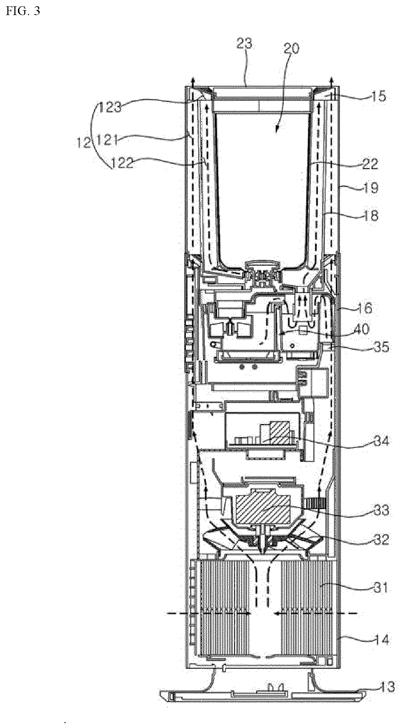

Referring to FIG. 3, the humidifier 1 will be described.

FIG. 3 is a vertical cross-sectional view of the humidifier 1.

The humidifier 1 may include a filter 31. The filter 31 may be disposed inside the intake grille 14. The filter 31 may be cylindrical.

The humidifier 1 may include a fan 32. The fan 32 may be disposed above the filter 31. The fan 32 may be disposed inside the intake grille 14.

The humidifier 1 may include a fan motor 33. The fan motor 33 may rotate the fan 32. The fan motor 33 may be disposed above the fan 32.

The humidifier 1 may include a controller 34. The controller 34 may be disposed inside the case 10. The controller 34 may be disposed between the fan motor 33 and a humidification assembly 40. The controller 34 may control the operation of the fan motor 33 and the humidification assembly 40. The controller 34 may include a plurality of PCB substrates.

The humidifier 1 may include an auxiliary fan 35. The auxiliary fan 35 may be disposed inside the case 10. The auxiliary fan 35 may be disposed at an entrance of the humidification assembly 40.

The humidifier 1 may include the humidification assembly 40. The humidification assembly 40 may be disposed inside the case 10. The humidification assembly 40 may be disposed between the water reservoir 20 and the fan 32. The humidification assembly 40 may cause water supplied from the water reservoir 20 to break up into tiny particles and deliver them to the discharge opening 12. Part of air blown by the fan 32 may enter the humidification assembly 40.

The discharge grille 15 may be disposed between the water reservoir 20 and the outer case 19. The inner case 18 may be disposed between the reservoir 20 and the outer case 19. The discharge grille 15 may be disposed above the inner case 18.

The discharge opening 12 may include a first discharge opening 121. The first discharge opening 121 may be formed between the outer case 19 and the inner case 18. The air blown by the fan 32 may flow upward through the first discharge opening 121. The first discharge opening 121 may extend in a ring shape. The first discharge opening 121 may be called "air flow path".

The discharge opening 12 may include a second discharge opening 122. The second discharge opening 122 may be formed between the inner case 18 and the water reservoir 20. Humidified air that has passed through the humidification assembly 40 may flow upward through the second discharge opening 122. The second discharge opening 122 may extend in a ring shape. The second discharge opening 122 may be called "humidification flow path".

The discharge opening 12 may include a mixing discharge opening 123. The mixing discharge opening 123 may be formed above the first discharge opening 121 and the second discharge opening 122. The mixing discharge opening 123 may be formed at the discharge grille 15. The mixing discharge opening 123 may extend in a ring shape. The mixing discharge opening 123 may be formed between the outer case 19 and the bucket cover 23. Air flowing in the first discharge opening 121 and air flowing in the second discharge opening 122 may be mixed together in the mixing discharge opening 123.

Conceptually, the discharge opening 12 may include the first discharge opening 121, the second discharge opening 122, and the mixing discharge opening 123. Then again, it may refer to a space open toward the outside of the case 10, separated from the above-mentioned components 121, 122, and 123. The discharge opening 12 may open toward the top of the case 10. The discharge opening 12 may be formed at the discharge grille 15. The discharge opening 12 may be formed to surround the water reservoir 20. The air blown by the fan 32 may flow through the discharge opening 12.

The air flow path 121 may be connected to the discharge opening 12. The humidification flow path 122 may be connected to the discharge opening 12. The air in the air flow path 121 and the air in the humidification flow path 122 may be mixed together in the discharge opening 12.

Air admitted into the case 10 through the intake grille 14 may pass through a filter 31 and be blown upward by the fan 32. Part of the air blown upward by the fan 32 may enter the humidification assembly 40, and the rest may flow upward through the first discharge opening 121. The air admitted to the humidification assembly 40, containing fine water drops, may flow upward through the second discharge opening 122. The air flowing through the first discharge opening 121 and the humidified air flowing through the second discharge opening 122 may be mixed together in the mixing discharge opening 123 and discharged upward of the humidifier 1.

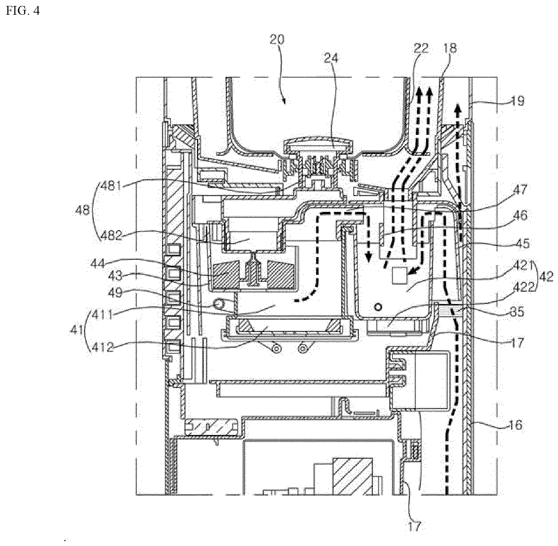

Referring to FIG. 4, the humidifier 1 will be described.

FIG. 4 is a partial vertical cross-sectional view of the humidifier 1.

The humidifier 1 may include the humidification assembly 40. The humidification assembly 40 may be disposed below the water reservoir 20. Humidified air produced in the humidification assembly 40 may be discharged upward.

The humidification assembly 40 may include a heating device 41. The heating device 41 may heat water and air admitted into it.

The heating device 41 may include a heating chamber 411. The heating chamber 411 may internally have a space. The water stored in the water reservoir 20 may be admitted to the heating chamber 411.

The heating device 41 may include a heater 412. The heater 412 may be attached to the bottom of the heating chamber 411. The heater 412 may apply heat into the heating chamber 411. The heater 412 may heat the water and air admitted into the heating chamber 411.

The humidification assembly 40 may include a humidification device 42. The humidification device 42 may convert the admitted water into fine water drops and discharge them upward.

The humidification assembly 42 may include a humidification chamber 421. The humidification chamber 421 may internally have a space. The water heated in the heating device 41 may be admitted to the humidification chamber 421.

The humidification device 42 may include a vibrating device 422. The vibrating device 422 may be attached to the bottom of the humidification chamber 421. The vibrating device 422 may generate vibrations by using ultrasonic waves. By the operation of the vibrating device 422, the water in the humidification chamber 421 may be converted into fine water drops. The humidification device 42 may work on the same principle as well-known ultrasonic humidifiers.

The humidification assembly 40 may include a valve housing 43. The valve housing 43 may be attached to the heating device 41. The valve housing 43 may be disposed within the heating chamber 411.

The humidification assembly 40 may include a valve 44. The valve 44 may be a floating valve. The valve 44 may be movably disposed inside the valve housing 43. The valve 44 may be moved upward and downward within the valve housing 43 and selectively supply water into the heating chamber 411.

The humidification assembly 40 may include an inlet 45. The inlet 45 may be connected to the humidification device 42. The inlet 45 may be connected to the inside of the humidification chamber 421. Part of the air blown by the fan 32 (see FIG. 3) may enter the humidification chamber 421 through the inlet 45. The auxiliary fan 35 may be attached to the inlet 45. The auxiliary fan 35 may draw air flowing between the outer case 16 and the inner case 17 into the inlet 45.

The humidification assembly 40 may include an outlet 46. The outlet 46 may be connected to the humidification device 42. The outlet 46 may protrude toward the inside of the humidification chamber 421. Fine water drops produced by the humidification device 42 may flow upward through the outlet 46.

The humidification assembly 40 may include a connector 47. The connector 47 may connect the heating device 41 and the humidification device 42. The connector 47 may connect the heating chamber 411 and the humidification chamber 421. The connector 47 may face the outlet 46.

The humidification assembly 40 may include a supply port 48. The supply port 48 may connect the water reservoir 20 and the heating device 41. The water in the water reservoir 20 may be admitted into the heating device 41 through the supply port 48.

The supply port 48 may include a first supply port 481. The first supply port 481 may be connected to the water reservoir 20. The first supply port 481 may be connected to the bottom of the bucket housing 22.

The water reservoir 20 may include a discharge port 24. The discharge port 24 may be disposed at the bottom of the water reservoir 20. The discharge port 24 may be connected to the first supply port 481.

The supply port 48 may include a second supply port 482. The second supply port 482 may connect the first supply port 481 and the valve housing 43. Water admitted into the first supply port 481 may be admitted into the valve housing 43 through the second supply port 482.

The humidification assembly 40 may include a connecting pipe 49. The connecting pipe 49 may connect the heating device 41 and the humidification device 42. The connecting pipe 49 may connect the heating chamber 411 and the humidification chamber 421. The water in the heating chamber 411 may be admitted into the humidification chamber 421 through the connecting pipe 49.

Part of the air flowing between the outer case 16 and the inner case 17 may be admitted to the humidification device 42 through the inlet 45. The air admitted to the humidification device 42, containing fine water drops produced within the humidification device 42, may flow upward through the outlet 46. The air flowing upward through the outlet 46 may flow upward between the water reservoir 20 and the inner case 18.

The water in the water reservoir 20 may be admitted into the valve housing 43 through the supply port 48. The water in the valve housing 43 may be admitted into the heating device 41 by movement of the valve 44. The water admitted into the heating device 41 may be heated by the heater 412. The water heated within the heating chamber 411 may be admitted into the humidification chamber 421 through the connecting pipe 49. The water admitted into the humidification chamber 421 may be converted into fine water drops by the vibrating device 422. The fine water drops, along with the air admitted through the inlet 45, may flow upward through the outlet 46.

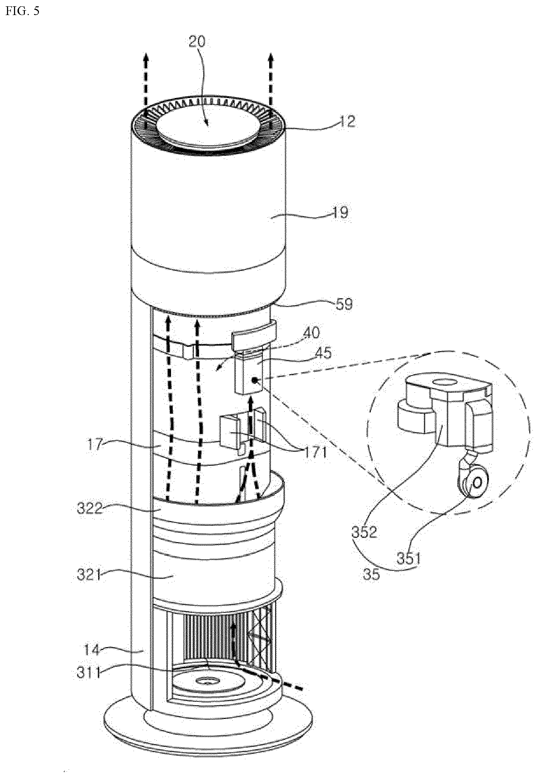

Referring to FIG. 5, the humidifier 1 will be described.

FIG. 5 illustrates a humidifier 1 from which the first outer case 16 is removed.

The humidifier 1 may include a filter mounting space 311. The filter mounting space 311 may be formed inside the intake grille 14. The filter 31 (see FIG. 3) may be disposed in the filter mounting space 311. The air admitted through the intake grille 14 may pass through the filter mounting space 311 and flow upward.

The humidifier 1 may include a fan housing 321. The fan 32 (see FIG. 3) may be disposed inside the fan housing 321. The fan housing 321 may be disposed above the filter 31 (see FIG. 3).

The humidifier 1 may include a housing top 322. The housing top 322 may form the top of the fan housing 321. The housing top 322 may be spaced radially outward from the inner case 17. The air blown by the fan 32 (see FIG. 3) may flow upward through a space formed between the inner case 17 and the housing top 322.

The humidifier 1 may include a guide 171. The guide 171 may protrude radially outward from the inner case 17. The guide 171 may be disposed between the inlet 45 and the fan housing 321. A plurality of guides 171 may be spaced apart from each other in a circumferential direction of the inner case 17. A space between the plurality of guides 171 may vertically face the inlet 45. A space between the plurality of guides 171 may vertically face the housing top 322.

The humidifier 1 may include an inner grille 59. The inner grille 59 may be disposed above the humidification assembly 40. The inner grille 59 may be disposed radially outward from the inner case 17. The inner grille 59 may be attached to the outer case 19.

Part of the air blown by the fan 43 (see FIG. 3) may enter the inlet 45. Part of the air blown by the fan 32 may enter the inlet 45 through the space between the plurality of guides 171. The air admitted to the humidification assembly 40 through the inlet 45, containing fine water drops, may be discharged through the discharge opening 12.

The auxiliary fan 35 may be disposed at the inlet 45. The auxiliary fan 35 may draw the air blown by the fan 32 (see FIG. 3) into the humidification assembly 40.

The auxiliary fan 35 may include a fan device 351. The fan device 351 may be a turbofan. The fan device 351 may be a sirocco fan.

The auxiliary fan 35 may include a supporter 352. The supporter 352 may be attached to the fan device 351. The supporter 352 may be fixed to the inlet 45. The supporter 352 may connect the fan device 351 and the inlet 45.

Part of the air blown by the fan 32 may flow upward through a space between the outer case 16 (see FIG. 3) and the inner case 17 and pass through the inner grille 59. The air that has passed through the inner grille 59 may flow upward and be discharged through the discharge opening 12.

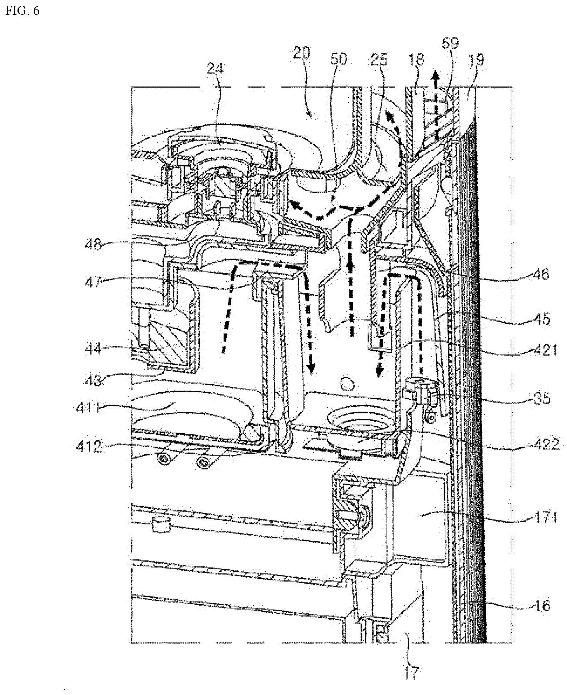

Referring to FIG. 6, the humidifier 1 will be described.

FIG. 6 is a vertical cutaway view of the humidifier 1 when viewed obliquely.

Part of air flowing upward between the outer case 16 and the inner case 17 may enter the inlet 45.

The auxiliary fan 35 may draw the air between the outer case 16 and the inner case 17 into the humidification device 42 through the inlet 45.

The guide 171 may vertically face the inlet 45. The guide 171 may be disposed below the auxiliary fan 35.

The air admitted into the humidification chamber 421 through the inlet 45 may be mixed with water drops produced by the operation of the vibrating device 422. The humidified air mixed with the water drops may flow upward through the outlet 46.

The humidification chamber 421 may communicate with the heating chamber 411. The connector 47 may connect the humidification chamber 421 and the heating chamber 411.

Air in the heating chamber 411 may be mixed with the air admitted into the humidification chamber 421 and released to the outlet 46. Part of the water admitted into the heating chamber 411 may evaporate and enter the humidification chamber 421 in the form of vapor. The vapor produced in the heating chamber 411 may be mixed with the humidified air in the humidification chamber 421 and released to the outlet 46.

The air flowing upward from the humidification chamber 421 through the outlet 46 may be admitted between the water reservoir 20 and the inner case 18. The humidified air discharged through the outlet 46 may flow upward between the water reservoir 20 and the inner case 18.

The humidifier 1 may include an inner cover 50. The inner cover 50 may be connected to the outlet 46. The inner cover 50 may be disposed between the water reservoir 20 and the humidification assembly 40. The air flowing through the outlet 46 may flow upward from the inner cover 50.

The water reservoir 20 may include a guide rim 25. The guide rim 25 may extend along the circumference of the water reservoir 20. The guide rim 25 may be spaced upward from the outlet 46. The guide rim 25 may be spaced upward from the inner cover 50. The air flowing through the outlet 46 may be dispersed in a circumferential direction of the water reservoir 20 by the guide rim 25. The humidified air dispersed in the circumferential direction of the water reservoir 20 may flow upward between the water reservoir 20 and the inner case 18.

Part of the air flowing between the outer case 16 and the inner case 17 may flow upward through the inner grille 59. The inner grille 59 may be disposed between the outer case 19 and the inner case 18. The air that has passed through the inner grille 59 may flow upward between the outer case 19 and the inner case 18.

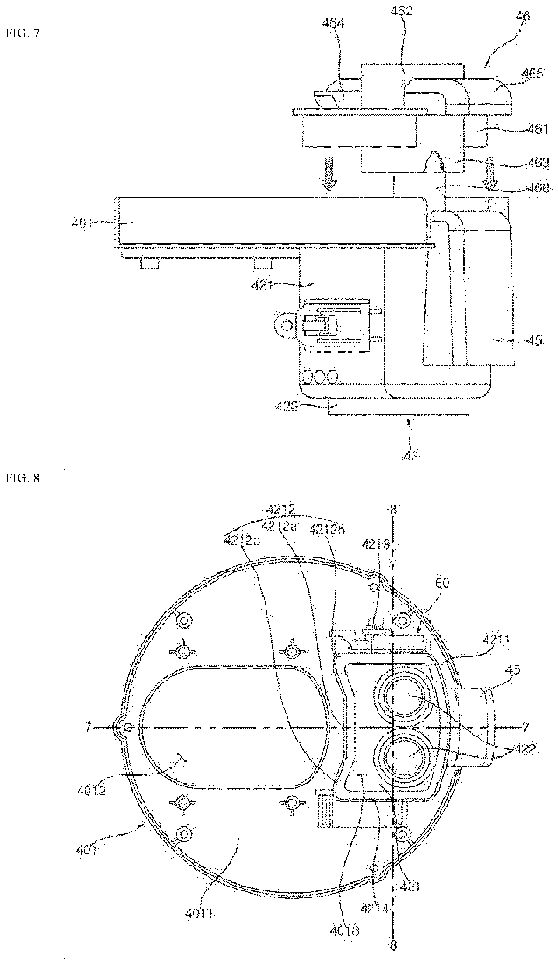

Referring to FIG. 7, the humidifier 1 will be described.

FIG. 7 depicts a separate view of part of the humidification assembly 40.

The humidification assembly 40 may include the humidification device 42. The humidification device 42 may produce mist. The humidification device 42 may include the humidification chamber 421 and the vibrating device 422.

The humidification assembly 40 may include a body 401. The body 401 may be cylindrical with an open top. The heating device 41 (see FIG. 4) and the humidification device 42 may be attached to the body 401. The heating device 41 (see FIG. 4) and the humidification device 42 may be attached to the underside of the body 401.

The humidification assembly 40 may include the outlet 46. Air admitted into the humidification device 42 through the inlet 45, which contains mist, may flow to the outlet 46. The outlet 46 may be attached to the body 401. The outlet 46 may be attached to the top of the humidification device 42.

The outlet 46 may include an outlet body 461. The outlet body 461 may be attached to the body 401. The outlet body 461 may be inserted into the humidification chamber 421.

The outlet 46 may include a mist discharge opening 462. The mist discharge opening 462 may protrude upward from the outlet body 461. Mist produced by the humidification device 42 may flow upward through the mist discharge opening 462.

The outlet 46 may include a mist intake opening 463. The mist intake opening 463 may protrude downward from the outlet body 461. The mist intake opening 463 may extend toward the inside of the humidification chamber 421. The mist produced by the humidification device 42 may flow toward the mist discharge opening 462 through the mist intake opening 463.

The outlet 46 may include a connector coupling portion 464. The connector coupling portion 464 may extend toward the heating device 41. The connector coupling portion 464 may be attached to the connector 47.

The outlet 46 may include an inlet cover 465. The inlet cover 465 may be disposed above the inlet 45. The inlet cover 465 may cover the top of the inlet 45.

The outlet 46 may include a supporter 466. The supporter 466 may extend downward from the outlet body 461. The supporter 466 may be disposed inside the humidification chamber 421. The supporter 466 may face a side wall of the humidification chamber 421.

Referring to FIG. 8, the humidifier 1 will be described.

FIG. 8 is a separate view of part of the humidification assembly 40 when viewed from above.

The body 401 may include a body plate 4011. The body plate 4011 may be disc-shaped. The humidification device 42 may be attached to the body plate 4011.

The body 401 may include a first opening 4012. The first opening 4012 may be formed through the body plate 4011. The heating device 41 (see FIG. 6) may be attached to the first opening 4012.

The body 401 may include a second opening 4013. The second opening 4013 may be formed through the body plate 4011. The humidification device 42 may be attached to the second opening 4013. The outlet 46 (see FIG. 7) may be inserted toward the inside of the humidification chamber 421 through the second opening 4013.

An internal space of the humidification chamber 421 may communicate with the second opening 4013. A plurality of vibrating devices 422 may be disposed.

The humidification chamber 421 may include an outer wall 4211. The outer wall 4211 may be attached to the inlet 45.

The humidification chamber 421 may include an inner wall 4212. The inner wall 4212 may be spaced from the outer wall 4211. The internal space of the humidification chamber 421 may be formed between the outer wall 4211 and the inner wall 4212. The inner wall 4212 may be positioned between the first opening 4012 and the outer wall 4211.

The inner wall 4212 may include a wall surface portion 4212a. The wall surface portion 4212a may face the outer wall 4211.

The inner wall 4212 may include a first edge portion 4212b. The first edge portion 4212b may extend outward from the wall surface portion 4212a. The first edge portion 4212b may be curved toward the center of the body 401.

The inner wall 4212 may include a second edge portion 4212c. The second edge portion 4212c may extend outward from the wall surface portion 4212a. The second edge portion 4212b may be curved toward the center of the body 401.

The wall surface portion 4212a may be disposed between the first edge portion 4212b and the second edge portion 4212c.

The humidification chamber 421 may include a first side wall 4213. The first side wall 4213 may connect the outer wall 4211 and the inner wall 4212. The first side wall 4213 may be connected to the first edge portion 4212b.

The humidification chamber 421 may include a second side wall 4214. The second side wall 4214 may connect the outer wall 4211 and the inner wall 4212. The second side wall 4214 may be connected to the second edge portion 4212c.

The first side wall 4213 and the second side wall 4214 may be spaced apart from each other. The internal space of the humidification chamber 421 may be formed between the first side wall 4213 and the second side wall 4214.

The humidifier 1 may include a sterilizer 60. The sterilizer 60 may be disposed in the humidification device 42. The sterilizer 60 may irradiate the humidification chamber 421 with ultraviolet light.

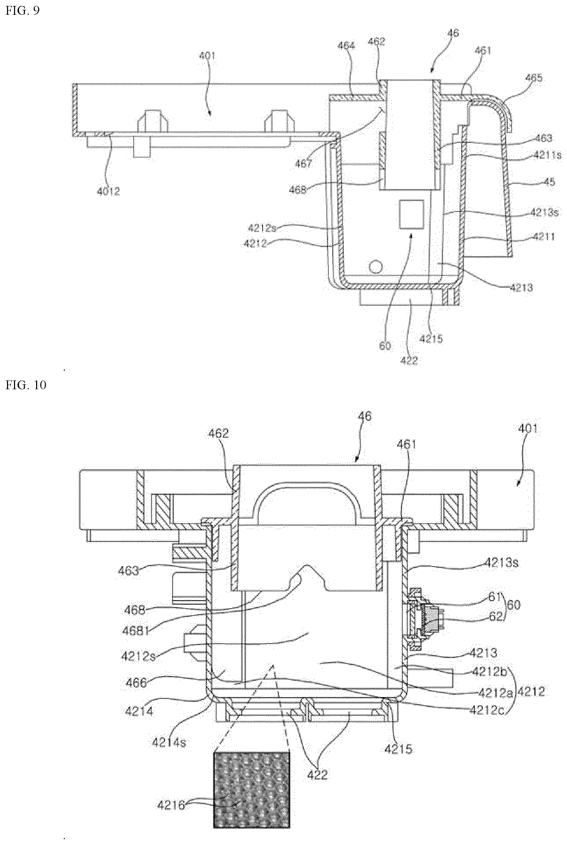

Referring to FIG. 9, the humidifier 1 will be described.

FIG. 9 is a vertical cross-sectional view of part of the humidification assembly 40 taken along the line 7-7 shown in FIG. 8.

The sterilizer 60 may be disposed on the first side wall 4213. The sterilizer 60 may be positioned between the outer wall 4211 and the inner wall 4212.

The humidification chamber 421 may include a chamber lower wall 4215. The chamber lower wall 4215 may be attached to the vibrating device 422. The vibrating device 422 may be disposed below the chamber lower wall 4215.

The sterilizer 60 may be positioned between the outlet 46 and the chamber lower wall 4215. The sterilizer 60 may be positioned lower than the outlet 46.

The outlet 46 may include the mist intake opening 463. The mist intake opening 463 may extend inward from the humidification chamber 421.

The outlet 46 may include a communicating opening 467. The communicating opening 467 may face the connector coupling portion 464. The communicating opening 467 may be formed through the mist intake opening 463.

The outlet 46 may include an outlet lower end 468. The outlet lower end 468 may be positioned inside the humidification chamber 421.

The sterilizer 60 may be positioned lower than the outlet lower end 468. The sterilizer 60 may be positioned higher than the chamber lower wall 4215.

The humidification assembly 40 may include a reflector 4211s, 4212s, 4213s, and 4214s. The reflector 4211s, 4212s, 4213s, and 4214s may face the internal space of the humidification chamber 421. The reflector 4211s, 4212s, 4213s, and 4214s may be disposed on the side walls 4211, 4212, 4213, and 4214 of the humidification chamber 421. Light emitted from the sterilizer 60 may hit the reflector 4211s, 4212s, 4213s, and 4214s and reflect off it.

The reflector 4211s, 4212s, 4213s, and 4214s may include an outer reflector 4211s. The outer reflector 4211s may be disposed on the outer wall 4211. The outer reflector 4211s may be attached to a side of the outer wall 4211 that faces the internal space of the humidification chamber 421.

The reflector 4211s, 4212s, 4213s, and 4214s may include an inner reflector 4212s. The inner reflector 4212s may be disposed on the inner wall 4212. The inner reflector 4212s may be attached to a side of the inner wall 4212 that faces the internal space of the humidification chamber 421.

The reflector 4211s, 4212s, 4213s, and 4214s may include a first side reflector 4213s. The first side reflector 4213s may be disposed on the first side wall 4213. The first side reflector 4213s may be attached to a side of the first side wall 4213 that faces the internal space of the humidification chamber 421.

The reflector 4211s, 4212s, 4213s, and 4214s may include a second side reflector 4214s (see FIG. 10). The second side reflector 4214s may be disposed on the second side wall 4214. The second side reflector 4214s may be attached to a side of the second side wall 4214 that faces the internal space of the humidification chamber 421.

Referring to FIG. 10, the humidifier 1 will be described.

FIG. 10 is a vertical cross-sectional view of part of the humidification assembly 40 taken along the line 8-8 shown in FIG. 8.

The sterilizer 60 may be disposed on the first side wall 4213. The sterilizer 60 may irradiate the internal space of the humidification chamber 421 with light.

The outlet 46 may include a recess 4681. The recess 4681 may be formed at a lower end of the outlet 46. The recess 4681 may be formed by indenting the outlet 46 upward.

The sterilizer 60 may be positioned lower than the outlet lower end 468. The sterilizer 60 may be positioned lower than the recess 4681.

The sterilizer 60 may include a lamp 61. The sterilizer 60 may include a light-emitting portion 62. The lamp 61 may be exposed to the internal space of the humidification chamber 421. The lamp 61 may irradiate the inside of the humidification chamber 421 with ultraviolet light emitted from the light-emitting portion 62. The light-emitting portion 62 may be disposed on an outer side of the humidification chamber 421. The light-emitting portion 62 may emit ultraviolet light toward the lamp 61.

The supporter 466 may extend into the humidification chamber 421. The supporter 466 may extend along the second side wall 4214. The supporter 466 may be spaced from the first side wall 4213. The supporter 466 may face the sterilizer 60. The supporter 466 may cover part of the second side wall 4214. The supporter 466 may be made of an opaque material. Then again, the supporter 466 may be made of a transparent material and reflect ultraviolet light.

The second side reflector 4214s may be exposed to the underside of the supporter 466. On the other hand, the second side reflector 4214s may be attached to one side of the supporter 466. In this case, light emitted by the sterilizer 60 may reflect off the second side reflector 4214s disposed on one side of the supporter 466.

The supporter 466 may face the second edge portion 4212c. Part of the supporter 466 may extend in a direction in which the second edge portion 4212c is curved.

The first edge portion 4212b may be positioned between the mist intake opening 463 and the first side wall 4213.

The humidification chamber 421 may include a dimple 4216. A plurality of dimples 4216 may be formed. The dimple 4216 may be formed by indenting the reflector 4211s, 4212s, 4213s, and 4214s in a direction away from the internal space of the humidification chamber 421.

Ultraviolet light emitted from the sterilizer 60 may be reflected off the reflector 4211s, 4212s, 4213s, and 4214s and diffused uniformly through the inside of the humidification chamber 421. In this case, the dimple 4216 may facilitate the diffusion of light by diversifying the angle of reflection of ultraviolet light.

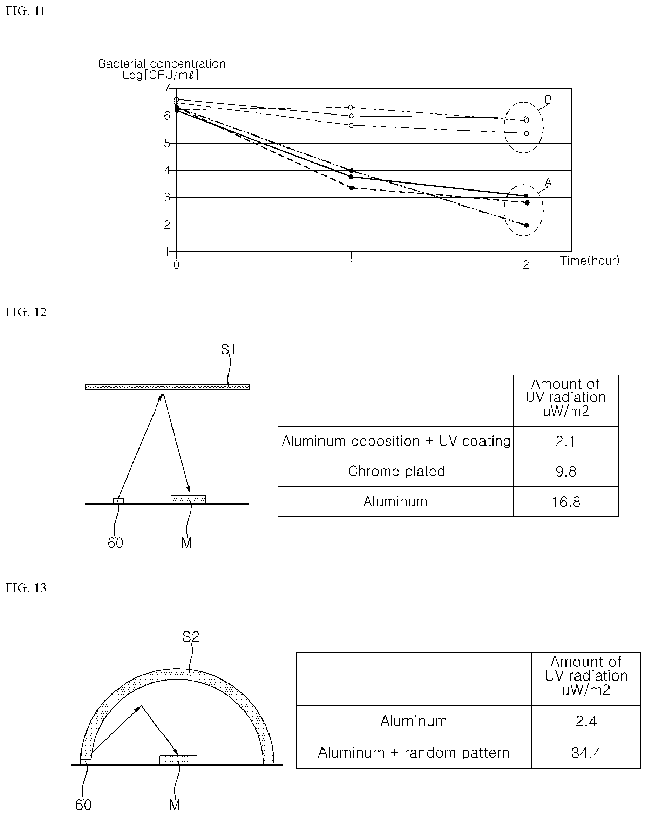

Referring to FIG. 11, the humidifier 1 will be described.

FIG. 11 is a graph comparing (A) the sterilization efficiency obtained when the reflector 4211s, 4212s, 4213s, and 4214s according to an embodiment of the present disclosure was employed and (B) the sterilization efficiency obtained when it was not employed.

It can be found out that, when the reflector 4211s, 4212s, 4213s, and 4214s was employed, the bacterial concentration in the humidification device 42 decreased further over time, compared to (B) when the reflector 4211s, 4212s, 4213s, and 4214s was not employed.

Referring to FIGS. 12 and 13, the humidifier 1 will be described.

FIG. 12 shows a comparison of the sterilization efficiency between different materials of a flat surface S1 when light was reflected off the flat surface S1. FIG. 13 shows a comparison of the sterilization efficiency obtained when light was reflected off a curve surface S2 and the sterilization efficiency obtained when light was reflected off a flat surface S1.

The table in FIG. 12 shows the amount of light radiation that reached a measuring instrument M when the sterilizer 60 reflected light off a flat-surface reflector S 1. Referring to the table, it can be found out that, when a reflector S1 with an aluminum surface was used, the amount of ultraviolet light reaching the measuring instrument M was largest.

The table in FIG. 13 shows the amount of light radiation that reached a measuring instrument M when the sterilizer 60 reflected light off a curved-surface reflector S2. Referring to the table, it can be found out that, when the curved-surface reflector S2 was used, the amount of ultraviolet light reaching the measuring instrument M was larger, compared to when the flat-surface reflector S1 was used, in which case both reflectors were made of the same material.

The reflector 4211s, 4212s, 4213s, and 4214s of this disclosure may be made of a chrome-plated material. The reflector 4211s, 4212s, 4213s, and 4214s of this disclosure may include a plurality of dimples 4216. Therefore, as shown in FIGS. 12 and 13, the amount of ultraviolet light reflected within the humidification chamber 421 may be increased.

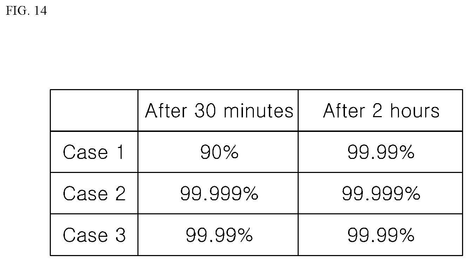

Referring to FIG. 14, the humidifier 1 will be described.

FIG. 14 is a table comparing the sterilization efficiency of a reflector 4211s, 4212s, 4213s, and 4214s depending on its material and whether or not it has dimples 4216.

Case 1 shows the sterilization efficiency over time for an aluminum reflector 4211s, 4212s, 4213s, and 4214s.

Case 2 shows the sterilization efficiency over time for an aluminum reflector 4211s, 4212s, 4213s, and 4214s with dimples 4216.

Case 1 shows the sterilization efficiency over time for a chrome-plated reflector 4211s, 4212s, 4213s, and 4214s with no dimples 4216.

Referring to the table, it can be found out that Case 2 using an aluminum reflector 4211s, 4212s, 4213s, and 4214s with dimples 4216 showed greatest sterilization efficiency.

The reflector 4211s, 4212s, 4213s, and 4214s according to an embodiment of this disclosure may be an aluminum reflector or a chrome-plated reflector.

It will be apparent that, although the exemplary embodiments have been shown and described above, the present disclosure is not limited to the above-described specific embodiments, and various modifications and variations can be made by those skilled in the art without departing from the gist of the appended claims. Thus, it is intended that the modifications and variations should not be understood independently of the technical concept or prospect of the present disclosure

The present disclosure may be embodied in various modifications, and its scope of rights is not limited by the foregoing embodiments. Thus, the modifications should be construed as falling within the scope of this disclosure as long as they include components as claimed in the claims of this disclosure.

Certain embodiments or other embodiments of the disclosure described above are not mutually exclusive or distinct from each other. Any or all elements of the embodiments of the disclosure described above may be combined with another or combined with each other in configuration or function.

For example, a configuration "A" described in one embodiment of the disclosure and the drawings and a configuration "B" described in another embodiment of the disclosure and the drawings may be combined with each other. Namely, although the combination between the configurations is not directly described, the combination is possible except in the case where it is described that the combination is impossible.

Although embodiments have been described with reference to a number of illustrative embodiments thereof, it should be understood that numerous other modifications and embodiments can be devised by those skilled in the art that will fall within the scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A humidifier comprising:
a case (10); and
a humidification assembly (40) disposed inside the case (10), the humidification assembly (40) is configured to produce mist, wherein the humidification assembly (40) includes:
a humidification chamber (421) with a space for holding water;
a sterilizer (60) configured to irradiate an inside of the humidification chamber (421) with light; and
a reflector (4211s, 4212s, 4213s. 4214s) disposed along a peripheral wall of the humidification chamber (421).

2. The humidifier of claim 1, wherein the reflector (4211s, 4212s, 4213s. 4214s) is spaced from the sterilizer (60) and disposed to face the sterilizer (60).

3. The humidifier of claim 1 or 2, wherein the humidification chamber (421) includes:
an inlet (45) through which air is admitted into the humidification chamber (421);
an outer wall (4211) attached to the inlet (45);
an inner wall (4212) spaced from the outer wall (4211); and
a first side wall (4213) connecting the outer wall (4211) and the inner wall (4212),
wherein the sterilizer (60) is disposed on the first side wall (4213).

4. The humidifier of claim 3, wherein the reflector (4211s, 4212s, 4213s. 4214s) includes:
an outer reflector (4211s) disposed on the outer wall (4211); and
an inner reflector (4212s) disposed on the inner wall (4212).

5. The humidifier of claim 3 or 4, wherein the humidification assembly (40) includes an outlet (46) through which mist produced in the humidification assembly (40) is discharged, and the humidification chamber (421) includes a second side wall (4214) spaced from the first side wall (4213),
wherein the outlet (46) includes a supporter (466) that extends downward toward the inside of the humidification chamber (421) and is disposed more adjacent to the second side wall (4214) than to the first side wall (4213).

6. The humidifier of claim 3, 4 or 5, wherein the inner wall (4212) includes:
a wall surface portion (4212a) spaced from the first side wall (4213);
a first edge portion (4212b) that connects the wall surface portion (4212a) and the first side wall (4213) and is curved from the wall surface portion (4212a) in a direction away from the inside of the humidification chamber (421); and
a second edge portion (4212c) being symmetrical to the first edge portion (4212b) with respect to the wall surface portion (4212a).

7. The humidifier of any one of the preceding claims, wherein the humidification assembly (40) includes:
an inlet (45) through which air is admitted into the humidification chamber (421); and
an outlet (46) through which mist produced in the humidification chamber (421) is discharged,
wherein the sterilizer (60) is spaced from both the inlet (45) and the outlet (46).

8. The humidifier of any one of the preceding claims, wherein the humidification assembly (40) includes:
a heating device (41) configured to heat water and to communicate with the humidification chamber (421); and
a body (401) including a first opening (4021) communicating with the heating device (41) and a second opening (4031) communicating with the humidification chamber (421),
wherein the sterilizer (60) is positioned lower than the second opening (4031).

9. The humidifier of any one of the preceding claims, wherein the humidification assembly (40) includes an outlet (46) through which mist produced in the humidification chamber (421) is discharged, wherein the sterilizer (60) is positioned lower than the outlet (46).

10. The humidifier of claim 9, wherein the outlet (46) includes:
an outlet lower end (468) positioned inside the humidification chamber (421); and
a recess (4681) formed by indenting the outlet lower end (468) upward,
wherein the sterilizer (60) is positioned lower than the recess (4681).

11. The humidifier of claim 9 or 10, wherein the outlet (46) includes:
an outlet body (461) attached to the top of the humidification chamber (421);
a mist discharge opening (462) protruding upward from the outlet body (461); and
a mist intake opening (463) protruding from the outlet body (461) into the humidification chamber (40),
wherein the sterilizer (60) is positioned lower than the mist intake opening (463).

12. The humidifier of any one of the preceding claims, wherein the humidification assembly (40) includes:
a heating chamber (411) internally having a space for heating water;
a connector (47) connecting the heating chamber (411) and the humidification chamber (421);
a mist inlet opening extending vertically toward the inside of the humidification chamber (421); and
a communicating opening (467) formed through the mist inlet opening, that opens toward the connector (47),
wherein the sterilizer (60) is positioned lower than the communicating opening (467).

13. The humidifier of any one of the preceding claims, wherein the reflector (4211s, 4212s, 4213s. 4214s) includes a dimple (4216) formed by indenting the reflector (4211s, 4212s, 4213s. 4214s) in a direction away from the internal space of the humidification chamber (421).

14. The humidifier of any one of the preceding claims, wherein the reflector (4211s, 4212s, 4213s. 4214s) is made of a chrome-plated material.

15. The humidifier of any one of the preceding claims, further comprising:
an intake opening (11) through which air is admitted into the case (10);
a discharge opening (12) through which air admitted through the intake opening (11) flows;
a humidification flow path (122) connecting the humidification assembly (40) and the discharge opening (12); and
a air flow path (121) separated from the humidification flow path (122) and connected to the discharge opening (12).
